# EUROPEAN PATENT APPLICATION

(11) **EP 2 911 169 A2**
(43) Date of publication of application: **26.08.2015**
(21) Application number: 14200336.7
(22) Date of filing: 24.12.2014
(51) Int. Cl.: H01H 9/04, A61B 5/117, G06K 9/00, H01H 13/06

(54) **Electronic device including physical key**

(30) Priority: 21.02.2014 KR 20140020903
(71) Applicant: Samsung Electronics Co., Ltd, Gyeonggi-do 443-742 (KR)
(72) Inventor: Choung, Tae-Doo, 443-742 Gyeonggi-do (KR); Lee, Seung-Hyun, 443-742 Gyeonggi-do (KR); Kwon, Taek-Soo, 443-742 Gyeonggi-do (KR)
(74) Representative: Birchenough, Lewis

(57) **Abstract**

Disclosed is an electronic device including a portable electronic housing. The electronic device also includes a pressible key provided on one surface of the housing. The electronic device further includes a fingerprint recognition sensor formed in the key to be at least partially exposed to a surface of the key. An uppermost surface of the fingerprint recognition sensor protrudes further than a surface of the housing surrounding the key.

## Description

### BACKGROUND OF THE INVENTION

### 1. Field of the Invention

The present disclosure relates to an electronic device, and more particularly to an electronic device including a physical key.

### 2. Description of the Related Art

Generally, the term "electronic device", for example a portable terminal, a Personal Media Player (PMP), and an MP3 player can refer to devices which a user carries and, with which, uses various contents. Portable electronic devices can be used during the mobility of a user because they have easy portability and various functions thereof. Such portable electronic devices can have various form factors according to a use, the trend of the times, or a consumer's requirement. Recently, a use of a bar-type electronic device such as a smart phone, a tablet PC and the like, which has various functions, has been increased. Improvements to electronic devices are needed.

### SUMMARY OF THE INVENTION

To address the above-discussed deficiencies, it is a primary object to provide an electronic device by which a user can easily contact a fingerprint recognition sensor and the recognition of the fingerprint of the user can be improved by protruding a front surface of the fingerprint recognition sensor integrally formed with a key further than a surface of a housing of the electronic device.

In a first example, an electronic device is provided by which a home key can be easily assembled by assembling the home key using an assembly jig employing an air-suction system such that a tolerance of the fingerprint recognition sensor between a key member and a decoration part of the fingerprint recognition sensor can be reduced.

In a second example, an electronic device is provided by which a damage to a flexible printed circuit board (FPCB) due to a high temperature can be prevented during an insert injection-molding of the FPCB, an additional insert injection-molding process and an additional waterproof member for waterproofing the FPCB can be excluded, and manufacturing costs can be reduced.

In a third example, a waterproof structure is provided by which a sealing member and an FPCB can be independently assembled without using an existing insert injection-molding process by modulating the sealing member and the FPCB into separate components by using first and second bonding parts, and accordingly, an assembly time of a product can be shortened and manufacturing costs can be further reduced.

In a fourth example, an electronic device is provided by which penetration of moisture introduced into an electronic device through an FPCB can be prevented by attaching the FPCB by using first and second bonding parts without using an existing insert injection-molding process and an waterproof member and penetration of moisture directly introduced into the electronic device can be additionally blocked by providing third and fourth bonding parts such that a housing (for example, a window and a bracket) and an FPCB can be attached to each other to be waterproofed.

In a fifth example, an electronic device is provided by which an FPCB can be replaced without damage when the electronic device is disassembled due to a failure, and can be easily reassembled by providing an opening/closing part for opening and closing the FPCB provided in the electronic device and a cap in a case of the electronic device.

In a sixth example, an electronic device is provided. The electronic device includes a portable electronic housing. The electronic device also includes a pressible key provided on one surface of the housing. The electronic device further includes a fingerprint recognition sensor formed in the key to be at least partially exposed to a surface of the key, wherein an uppermost surface of the fingerprint recognition sensor protrudes further than a surface of the housing surrounding the key.

In a seventh example, the sensor includes a living body recognition sensor. The living body recognition sensor includes any sensor other than the fingerprint recognition sensor. The sensor can include a grip sensor, a proximity sensor, or a gesture sensor.

In an eighth example, a method of manufacturing an electronic device is provided. The method includes manufacturing a decoration part of a key. The method also includes assembling the decoration part in an assembly jig to which an air-suction system having a plurality of air suction pipes are applied after overturning the decoration part. The method further includes fixing the decoration part by using the air suction pipes. The method includes assembling a key member of a fingerprint recognition sensor in the overturned decoration part and reducing an assembly tolerance between the decoration part and the key member by using the air suction pipes. The method also includes applying an adhesive to the decoration part and an upper end surface of the key member. The method further includes attaching a support member to the decoration part and an upper portion of the key member by using the adhesive.

In a ninth example, an electronic device is provided. The electronic device includes a portable electronic housing. The electronic device also includes a through-hole formed at a portion of one surface of the housing. The electronic device further includes a pressible key exposed through the through-hole of the housing. The electronic device includes a key switch located within the housing and configured to detect pressing of the key. The electronic device also includes a fingerprint recognition sensor provided within the key and exposed to an upper surface of the key. The electronic device further includes a flexible member inserted between the key and the key switch.

In a tenth example, the electronic device includes a waterproof structure extending from an inner surface of the housing around the through-hole and connected to the flexible member to surround the key within the housing.

In an eleventh example, the key includes an FPCB electrically connected to the sensor. The FPCB extends from a portion of the key and is disposed to pass through an opening formed in the waterproof structure.

In a twelfth example, an electronic device is provided. The electronic device includes a portable electronic housing. The electronic device also includes a sealing member located within the housing and having a through-portion. The electronic device further includes a first bonding part provided in the through-portion. The electronic device includes an FPCB attached to the first bonding part and coupled to the through-portion of the sealing member. The electronic device also includes a second bonding part provided between the housing and the sealing member, wherein a front surface of the FPCB is attached to the housing by the second bonding part and a rear surface of the FPCB is attached to the through-portion by the first bonding part.

In a thirteenth example, an electronic device is provided. The electronic device includes a housing. The electronic device also includes a case coupled to the housing. The electronic device further includes an FPCB provided between the housing and the case. The electronic device also includes an opening/closing part provided in the case. The electronic device further includes a cap detachably coupled to the opening/closing part, for opening and closing the FPCB.

In a fourteenth example, an electronic device is provided. The electronic device includes a portable electronic housing. The electronic device also includes a case coupled to the housing. The electronic device further includes an FPCB provided between the housing and the case and having a connector at one end thereof and a key at an opposite end thereof. The electronic device includes an opening/closing part provided in the case. The electronic device also includes a cap detachably coupled to the opening/closing part, for opening and closing the connector.

In a fifteenth example, a finger of a user can easily contact a home key and accordingly a fingerprint of the user can be detected better by protruding a front surface of a fingerprint recognition sensor integrally provided with a key further than a surface of a housing of the electronic device.

In a sixteenth example, a tolerance due to a height difference of upper end surfaces of a key member and a decoration part can be minimized and a key can be easily assembled by providing an assembly jig employing an air-suction system such that a height difference of the upper end surfaces of the key member and the decoration part of the fingerprint recognition part can be reduced when they are assembled.

In a seventeenth example, penetration of moisture introduced from the outside through a through-portion through which an FPCB penetrates to be coupled to the through-portion can be prevented by stacking first and second bonding parts and the FPCB to attach the housing and the sealing member of the electronic device, and damage of the FPCB generated during an insert injection-molding can be prevented without using an existing insert injection-molding process and an additional waterproof structure and manufacturing costs can be reduced.

In an eighteenth example, a sealing member and an FPCB can be independently assembled without using an existing insert injection-molding process by modulating the sealing member and the FPCB into separate components by using first and second bonding parts, and accordingly, an assembly time of a product can be shortened and manufacturing costs can be further reduced.

In a nineteenth example, damage to an FPCB generated when an electronic device is disassembled and assembled can be prevented by providing an opening part and a cap for opening/closing the FPCB of an electronic device to a case of the electronic device, and the FPCB can be replaced and reassembled without damage.

Before undertaking the DETAILED DESCRIPTION below, it may be advantageous to set forth definitions of certain words and phrases used throughout this patent document: the terms "include" and "comprise," as well as derivatives thereof, mean inclusion without limitation; the term "or," is inclusive, meaning and/or; the phrases "associated with" and "associated therewith," as well as derivatives thereof, may mean to include, be included within, interconnect with, contain, be contained within, connect to or with, couple to or with, be communicable with, cooperate with, interleave, juxtapose, be proximate to, be bound to or with, have, have a property of, or the like; and the term "controller" means any device, system or part thereof that controls at least one operation, such a device may be implemented in hardware, firmware or software, or some combination of at least two of the same. It should be noted that the functionality associated with any particular controller may be centralized or distributed, whether locally or remotely. Definitions for certain words and phrases are provided throughout this patent document, those of ordinary skill in the art should understand that in many, if not most instances, such definitions apply to prior, as well as future uses of such defined words and phrases.

### BRIEF DESCRIPTION OF THE DRAWINGS

For a more complete understanding of the present disclosure and its advantages, reference is now made to the following description taken in conjunction with the accompanying drawings, in which like reference numerals represent like parts:
FIG. 1 is an example perspective view showing a front surface of an electronic device according to this disclosure;
FIG. 2 is an example perspective view showing a rear surface of the electronic device according to this disclosure;
FIG. 3 is an example side sectional state showing a coupled state of the electronic device according to this disclosure;
FIG. 4 is an example plan view showing a signal pattern part of a fingerprint recognition sensor unit of the electronic device according to this disclosure;
FIG. 5 is an example side sectional view showing a home key of the electronic device according to this disclosure;
FIG. 6A is an example side sectional view showing an assembly process of a decoration part of the home key of the electronic device according to this disclosure;
FIG. 6B is an example side sectional view showing an assembly process of a key member and the decoration part of the electronic device according to this disclosure;
FIG. 6C is an example side sectional view showing a state in which a support member is assembled in the key member and the decoration part of the electronic device according to this disclosure;
FIG. 7 is an example flowchart showing a method of manufacturing a home key of an electronic device according to this disclosure;
FIG. 8 is an example exploded perspective view showing a configuration of a waterproof structure for waterproofing an electronic device according to this disclosure;
FIG. 9 is an example enlarged exploded perspective view of part A of FIG. 8 according to this disclosure;
FIG. 10 is an example enlarged side sectional view showing a movement passage of a movement part of the waterproof structure for waterproofing the electronic device according to this disclosure;
FIG. 11 is an example enlarged side sectional view showing a movement positioning part of the movement part of the waterproof structure for waterproofing the electronic device according to this disclosure;
FIG. 12 is an example side view showing a coupled state of the waterproof structure for waterproofing the electronic device according to this disclosure;
FIG. 13 is an example side view showing an in-use state of the waterproof structure for waterproofing the electronic device according to this disclosure;
FIG. 14 is an example enlarged side sectional view of part 310 of FIG. 13 according to this disclosure;
FIG. 15 is an example side view showing a coupled state of the waterproof structure for waterproofing the electronic device according to this disclosure;
FIG. 16 is an example enlarged side sectional view of part 312 of FIG. 15 according to this disclosure;
FIG. 17 is an example exploded perspective view showing an electronic device according to this disclosure;
FIG. 18 is an example perspective view showing a state in which an FPCB is coupled to a window of the electronic device according to this disclosure;
FIG. 19 is an example perspective view showing a coupled state of the electronic device according to this disclosure;
FIG. 20 is an example perspective view showing an opening/closing part of the electronic device according to this disclosure; and
FIG. 21 is an example perspective view showing an operational state of a cap part of the electronic device according to this disclosure.

### DETAILED DESCRIPTION

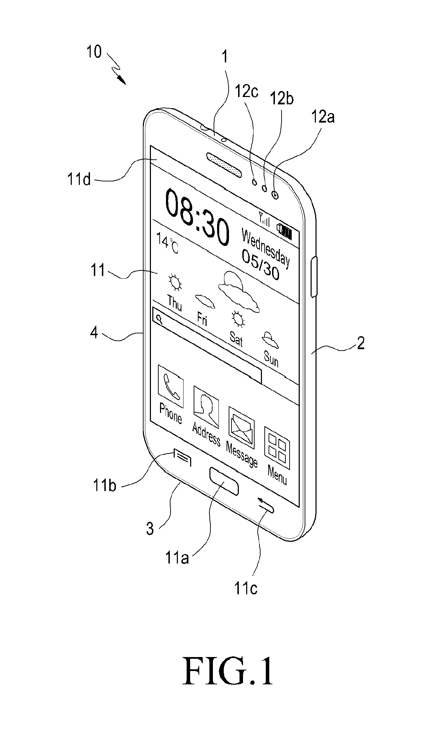

FIGURES 1 through 21, discussed below, and the various embodiments used to describe the principles of the present disclosure in this patent document are by way of illustration only and should not be construed in any way to limit the scope of the disclosure. Those skilled in the art will understand that the principles of the present disclosure may be implemented in any suitably arranged electronic device. Hereinafter, various embodiments of the present disclosure will be described in detail. Firstly, terms used in the various embodiments of the present disclosure will be briefly described.

With respect to the terms in the various embodiments of the present disclosure, the general terms which are currently and widely used are selected in consideration of functions of structural elements in the various embodiments of the present disclosure. However, meanings of the terms may be changed according to an inventor's intention, a judicial precedent, appearance of a new technology, and the like. In addition, at a certain case, a term arbitrarily selected by the applicant may be used. In such a case, the meaning of the term will be described in detail at the corresponding part in the description of the present disclosure. Thus, the terms used in various embodiments of the present disclosure should be defined based on the meanings of the terms and the overall contents of the embodiments of the present disclosure instead of simple titles of the terms.

Although the terms including an ordinal number such as first, second, etc. can be used for describing various elements, the structural elements are not restricted by the terms. The terms are only used to distinguish one element from another element. For example, without departing from the scope of the present disclosure, a first structural element may be named a second structural element. Similarly, the second structural element also may be named the first structural element.

In the case of a recent bar-type electronic device, as diary or multimedia function has been mounted on the electronic device, only the portable electronic device can be used to utilize various contents, including voice communication, a financial service, a game/multimedia service, a wireless Internet service, and the like. Electronic devices, such as smart phones, which have been widely used recently, can have various functions, and because a lot of personal information, for example, personal information related to banking or information related to payments in addition to simple personal information, can be stored in the electronic devices, various locking devices have been suggested. For example, a locking on/off of the electronic device can be controlled through a pattern provided to the touch panel, or a combination of numerals. In addition, in recent years, fingerprint recognition sensors have been applied to electronic device.

However, it can be difficult to mount a fingerprint recognition sensor in a narrow mounting area of a key switch installed to be pressed, in order to improve security and utility.

That is, in the electronic devices, it can be difficult to ensure a separate mounting space for mounting the fingerprint recognition sensor and security, and convenience may not be provided together.

In addition, the fingerprint recognition sensor can include a Flexible Printed Circuit Board (hereinafter, referred to as "FPCB") to be electrically connected to other components of the electronic device. A waterproof member formed of rubber or silicon through insert injection-molding can be provided in the FPCB to waterproof the FPCB. That is, the FPCB including the waterproof member can be attached and coupled to the case of the electronic device through interference-fitting, and accordingly moisture introduced into the electronic device through the FPCB can be prevented.

Because the existing FPCB can be configured such that the FPCB is positioned in an injection-molding mold to provide the FPCB with a waterproof member, and the waterproof member is formed through injection-molding, the FPCB can be damaged due to a high temperature in the interior of the mold during injection-molding. Further, a waterproof member can be additionally provided, increasing manufacturing costs.

A waterproof structure of an electronic device will be described. The electronic device can include all information and communication devices and multimedia devices, such as a Portable Multimedia Player (PMP), an MP3 player, a navigation, a game player, a notebook, a netbook, an advertising panel, a TV, a digital broadcasting receiver, a Personal Digital Assistant, and a smart phone, as well as all kinds of mobile communication terminals which operate according to communication protocols corresponding to various communication systems, and application devices thereof.

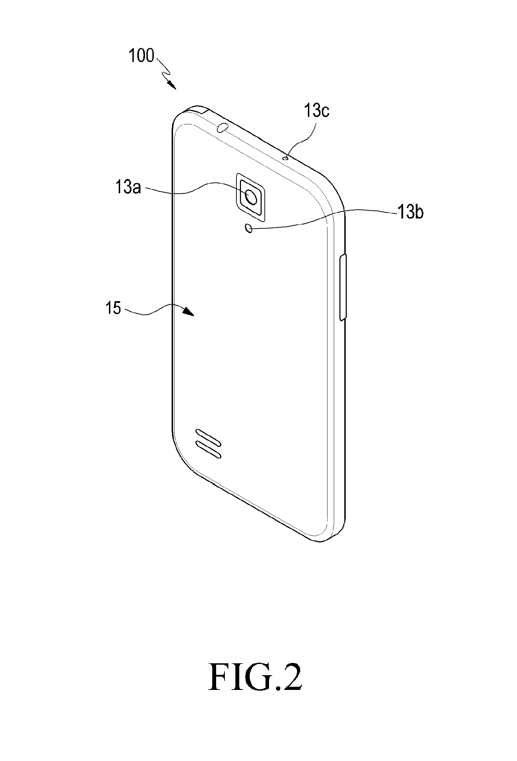

FIG. 1 is an example perspective view showing a front surface of the electronic device according to this disclosure. FIG. 2 is an example perspective view showing a rear surface of the electronic device according to this disclosure. The electronic device can be a smart phone or a tablet PC. Hereinafter, a configuration of an electronic device such as a smart phone will be described with reference to FIGS. 1 and 2. A touch screen 11 can be disposed at the center of a front surface of the electronic device 10. The touch screen 11 can occupy a great part of the front surface of the electronic device 10. FIG. 1 shows an example in which a main home screen is displayed on the touch screen 11. The main home screen can be the first screen displayed on the touch screen 11 when a power source of the electronic device 10 is switched on. When the electronic device 10 has different home screens of several pages, the main home screen can be the first home screen of the home screens of several pages. Short key icons for executing frequently used applications, main menu conversion keys, time, weather, and the like can be displayed on the home screen. The main menu conversion keys can display a menu screen on the touch screen 11. A status bar 11d for displaying statuses such as a battery charging state, an intensity of a received signal, and a current time can be formed at an upper end of the touch screen 11. A home key 11 a, a menu button 11b, and a back button 11c can be formed at a lower portion of the touch screen 11.

A main home screen on the touch screen 11 can be displayed on the touch screen 11 through the home key 11 a. For example, when the home button 11 a is touched in a state where the home screen is different from the main home screen or the menu screen is displayed, the main home screen can be displayed on the touch screen 11. If the home key 11 a is touched while applications are executed on the touch screen 11, the main home screen can be displayed on the touch screen 11. In addition, the home key 11a can be used to display recently used applications or a task manager on the touch screen 11. The menu button 11b can provide a connection menu that can be used on the touch screen 11. The connection menu can include a widget addition menu, a background changing menu, a search menu, an editing menu, and an environment setup menu. The back button 11c can display the screen which was executed shortly before the currently executed screen or can terminate the most recently used application. The home key 11 a can be installed together with an electronic component such as a fingerprint recognition sensor to receive a fingerprint input of the user. Various electronic components having functions heart-rate monitoring (HMR) as well as fingerprint recognition can be installed to be utilized as functional keys.

A first camera 12a, an illumination intensity sensor 12b, or a proximity sensor 12c can be provided in an upper end area of a front surface of the electronic device 10. A second camera 13a, a flash 13b, or a speaker 13c can be provided on a rear surface of the electronic device 100. If the electronic device 10 is configured such that a battery pack can be attached to or detached from the electronic device 10, a bottom surface of the electronic device 10 can be a battery cover 15 that is detachable.

The electronic devicecan be configured such that the battery pack is detachable and the battery cover can be coupled to or removed from the electronic device.

The home key 11a including the fingerprint recognition sensor can be utilized as a function key for driving the electronic device at the same time when the home key 11a is pressed and can perform a click operation as well.

Referring to FIG. 1, an edge located at the uppermost end when the electronic device 10 can be viewed from the front side and can have a small length is a first side 1, and an edge which is longer than the first side and disposed in a direction perpendicular to the first side 1 can be a second side 2.

An edge having the same length as that of the first side 1 and parallel to the first side 1 can be a third side 3, and an edge having the same length as that of the second side 2 and parallel to the second side can be a fourth side 4.

Referring to FIG. 1, the fingerprint recognition sensor 11b can have a length extending in a direction parallel to the third side and a width perpendicular to the lengthwise direction, and the length can be larger than the width.

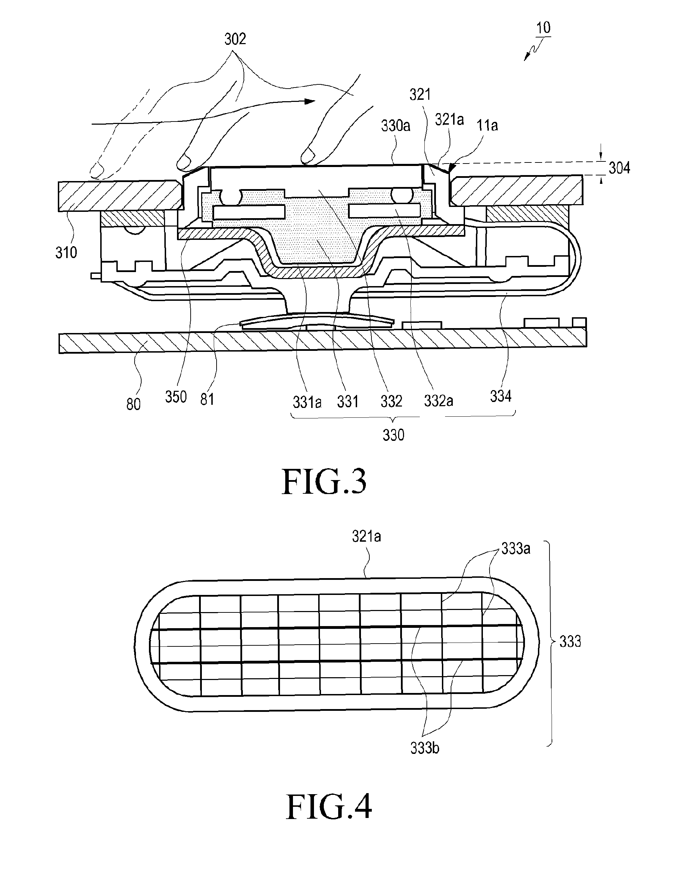

First, FIG. 3 is an example side sectional view showing a configuration of the electronic device 10 according to this disclosure.

Referring to FIG. 3, the home key 11a which can include a fingerprint recognition sensor 330 of the constituent components of the electronic device 10 will be described as an example. In more detail, the electronic device 10 can include a housing 310, a home key 11a, and a fingerprint recognition sensor 330.

The housing 310 can include a window and a bracket, and the home key 11a can be provided on one surface of the window. The home key 11a can be provided on one surface of the housing 310 such that a fingerprint contact of the user is easily made. The fingerprint recognition sensor 330 can be provided integrally with the home key 11a to detect a fingerprint of the user.

In an embodiment, because the front surface of the fingerprint recognition sensor 330 can protrude further than a surface of the housing 310, a fingerprint contact of the user can be easily made to increase the in-use efficiency of the user.

For example, a protruding area 304 can be formed between a front surface of the fingerprint recognition sensor 330 and the surface of the window to protrude from the surface of the window by a predetermined height.

The fingerprint recognition sensor 330 can be achieved in a swipe method or an area method. Of course, the fingerprint recognition sensor 330 can adopt a sensor of another type for recognizing a fingerprint of the user in addition to the swipe type or the area type.

For example, the fingerprint recognition sensor 330 of a swipe type can detect a fingerprint of the user when a finger 302 of the user is slid from the sensor, and the fingerprint recognition sensor 330 of an area type can detect a fingerprint of the user by touching the entire finger 302 of the user.

In the embodiment, the fingerprint recognition sensor 330 of a swipe type will be described herein.

In an embodiment, any one of a decoration part 321 (for example, a ring, a peripheral member, a decoration member) which is electrically isolated and a nonmetallic decoration part 321 can be provided around the home key 11a.

The decoration part 321 can have an inclined surface 321a or a convexly curved surface such that a finger of the user is easily slid when a fingerprint of the user contacts or approaches the fingerprint recognition sensor 330. When viewed from an upper side of one surface of the electronic device 10, the decoration part 321 can have a ring at least partially surrounding the home key 11a. The ring 321 can include a metallic material, and the ring 321 can be electrically isolated from the fingerprint recognition sensor 330. However, the material of the ring 321 may not be limited to the metallic material, but can be a nonmetallic material. The ring 321 can have at least one plane, at least one curved surface, or a combination of at least one plane and at least one curved surface which extends from a surface of the housing 310 to an uppermost surface of the fingerprint recognition sensor 330.

Here, a configuration of the fingerprint recognition sensor 330 will be described in more detail.

First, FIG. 3 is an example side sectional view showing a configuration of the home key 11a including the fingerprint recognition sensor 330 according to this disclosure, and FIG. 4 is an example plan view showing a signal pattern part 333 of the home key 11a according to this disclosure.

As shown in FIGS. 1 and 2, the fingerprint recognition sensor 330 can include a key member 331, a fingerprint recognition sensor 332, and a signal pattern part 333, or an FPCB 334. The key member 331 can have a substrate 332a, and can be assembled with the decoration part 321. The substrate 332a can have the signal pattern part 333, and can be provided in the key member 331 to be electrically connected to the FPCB 334. The signal pattern part 333 can be provided in the substrate 332a to generate a transmission (Tx) signal or a reception (Rx) signal according to a fingerprint contact of the user. The FPCB 334 can be electrically connected to the signal pattern part 333 and can be electrically connected to another electronic component provided in the electronic device 10 at the same time.

As in FIG. 4, the signal pattern part 333 can include a plurality of transmission (Tx) lines 333a and a plurality of reception (Rx) lines 333b to detect a fingerprint contact of the user when the signal pattern part 333 contacts the fingerprint contact of the user. The number of reception (Tx) lines 333b can be smaller than the number of transmission lines 333a.

An operation of the home key 11a including the fingerprint recognition sensor 330 will be described in more detail.

The home key 11 a can protrude from a portion of the housing 310 of the electronic device 10 and can be exposed to the outside at the same time. In this state, the finger 302 of the user can be slid along a surface of the housing 310, and the finger 302 of the user can contact the inclined surface 321a or the convexly curved surface of the decoration part 321 of the home key 11a first and the finger 302 can rise by a protruding area 304 of the home key 11a. The rising finger 302 can be slid on an upper surface of the fingerprint recognition sensor 330 of the home key 11a.

When the signal pattern part 333 of the fingerprint recognition sensor 330 contacts a fingerprint of the finger 302 of the user, the plurality of transmission (Tx) lines 333a and the plurality of reception (Rx) lines 333b can detect it and can generate transmission (Tx) signals and reception (Rx) signals. The signal pattern part 333 can bring the transmission (Tx) signals or the reception (Rx) signals into an image, and the fingerprint image can be transmitted to the controller. The controller can compare the fingerprint image with a fingerprint image of the user, which is stored in advance, and can recognize the user based on the comparison result.

For example, the signal pattern part 333 can detect and signalizes a fingerprint input by the plurality of transmission (Tx) lines 333a and the plurality of reception (Tx) lines 333b. The signal pattern part 333 can include a fingerprint detection sensor including the plurality of transmission (Tx) lines 333a and the plurality of reception (Rx) lines 333b, and the fingerprint detection sensor can be provided in the substrate 332a of the fingerprint recognition sensor. The signal pattern part 333 can be electrically connected to the controller by the FPCB 334. In addition, the fingerprint detection sensor can receive electric power through a power source provided in the electronic device 10.

The home key 11a can perform a click operation as in a mechanical button key, and the home key 11a can include a sensor other than the fingerprint recognition sensor 330. For example, a sensor such as a grip sensor, a proximity sensor, or a gesture sensor can be applied.

The home key 11 a can be electrically connected to the controller provided in the electronic device 10 and a power source by the FPCB 334.

For example, the controller can receive a fingerprint image generated in the signal pattern part 333, can compare the fingerprint image with a finger print image of the user stored in a memory in advance, and can recognize the user based on the comparison result. The power source can include a battery for supplying electric power to various components of the electronic device 10 under the control of the controller, and can perform a function of applying electric power output from a battery. The power source can supply the electric power supplied from the battery to the fingerprint recognition sensor 330 through a power supply line provided in the FPCB.

For example, when the user raises and positions the finger 302 on the protruding fingerprint recognition sensor 330 and slides the finger 302 from an upper side to a lower side or from a lower side to an upper side, the signal pattern part 333 can detect the fingerprint of the user, generate a transmission (Tx) signal and a reception (Rx) signal to being the fingerprint into an image, and transmit the fingerprint image to the controller. The controller can compare the transmitted fingerprint image with the fingerprint image of the user stored in the memory in advance, and can recognize the user based on the comparison result and can realize an operation in various modes of the electronic device 10. For example, a voice or image communication mode operation, a camera mode operation, and general operations of the electronic device can be realized. That is, various contents such as a financial business, a game/multimedia service, and a wireless Internet service as well as voice or image communications can be used through the electronic device.

In an embodiment, a coating layer 330a can be provided on an upper end surface of the fingerprint recognition sensor 330 to cover the substrate 332a. The coating layer 330a can be formed of a paint layer to protect the substrate 332a or the fingerprint recognition sensor 330, continue to detect a fingerprint, and make an external appearance of the electronic device appealing. The paint layer can be formed of various color paint layers.

The home key 11a will be described in more detail below with reference to the accompanying drawings.

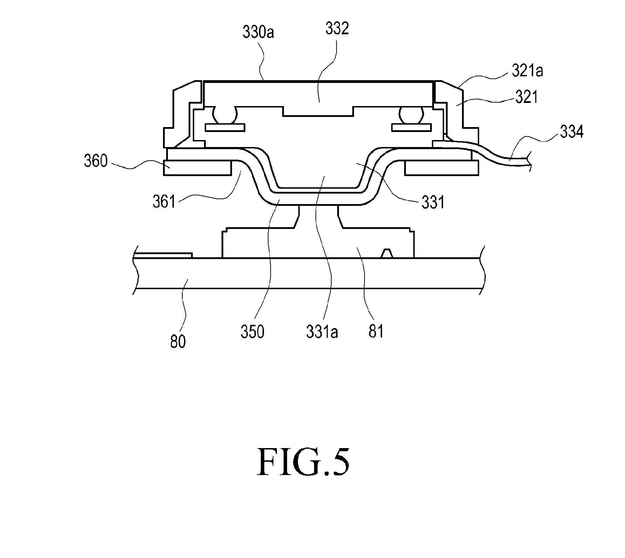

FIG. 5 is an example side sectional view showing the home key 11a of the electronic device 10 according to this disclosure.

First, referring to FIG. 5, a key actuator 331a coupled to the home key 11a and formed at a lower portion of the key member 331 and a base member 360 having a through-hole 361 through which a support member 350 can be provided at a lower portion of the home key 11a to face a key switch 81 provided in the printed circuit board 80 in the electronic device. However, the present disclosure is not limited to the through-hole formed in the base member but the base member can be blinded without using the through-hole. That is, the base member can have the through-hole 361 in various modifications as long as it includes the home key 360.

For example, the through-hole 361 of the base member 360 can couple and protrude the key actuator 331a and the support member 350 provided at a lower portion of the home key 11a such that the key actuator 331a and the support member 350 can face a boss provided on an upper surface of the key switch 81.

In an embodiment, in order to prevent damage to the fingerprint recognition sensor 332 when an impact is applied from an outside of the electronic device to the fingerprint recognition sensor 332 mounted within the home key 11a, the support member 350 having a high strength can support the home key 11a. The home key can be a pressible key, and can include a fingerprint recognition sensor by which a fingerprint can be recognized. When the user scrubs a surface of the home key 11a for recognition of a fingerprint, a key switch can be pressed on a rear surface of the home key 11a by a force of a finger of the user. The key switch can be replaced by a component operated by a force higher than an existing force. Because the existing key actuator 331a can be formed of a soft material (for example, silicon and urethane), it can be difficult to operate the key switch and the actuator can be damaged by a repeated operation. In order to prevent this, the key switch can be pressed by using the support member 350 having a high strength.

The support member 350 can be manufactured of an SUS material. Although the support member 350 can be formed, for example, of an SUS material for strength, the present disclosure is not limited thereto. That is, the support member 350 can be formed of various materials as long as the materials can ensure strength. For example, the material of the support member 350 can be a metal (for example, tungsten, aluminum, magnesium, zinc, and the like).

In an embodiment, because a metallic support member 350 can be coupled to the key actuator 331 a at a lower portion of the home key 11a while surrounding the key actuator 331 a, the strength of the home key 11 a including the fingerprint recognition sensor 330 can be improved and thus a function of the home key 11a can be improved.

In an embodiment, because the base member 360 having a through-hole 361 through which the support member 350 and the key actuator 331a pass is formed in the home key 11a, the support member 350 can directly face an upper surface of the key switch 81 such that the thickness of the home key 11a is reduced and the home key 11a is miniaturized.

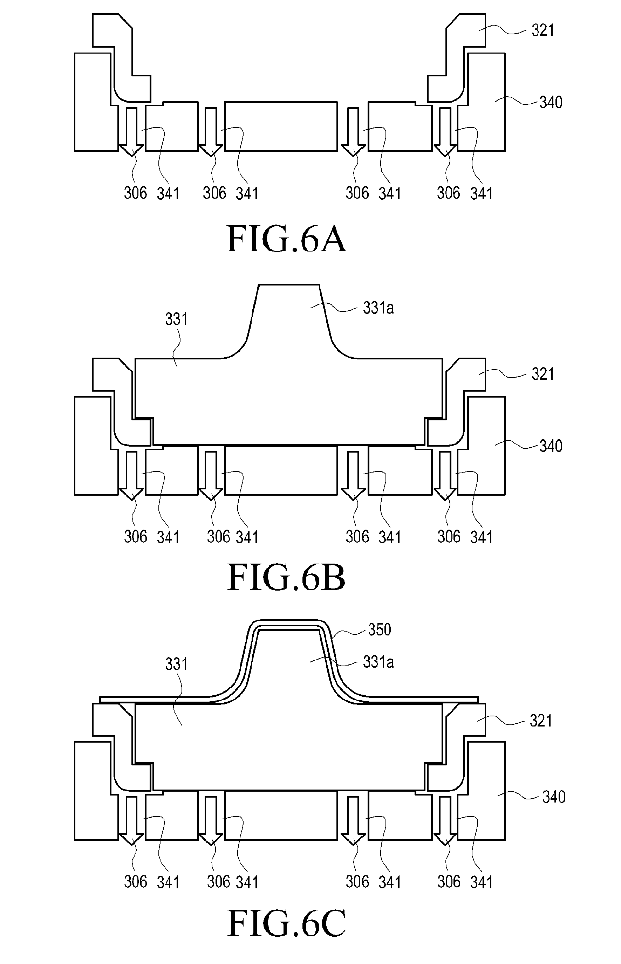

FIG. 6A is an example side sectional view showing an assembly process of the home key 11 a and the decoration part 321 of the electronic device 10 according to this disclosure. FIG. 6B is an example side sectional view showing an assembly process of the key member 331 and the decoration part 321 of the electronic device 10 according to this disclosure. FIG. 6C is an example side sectional view showing a state in which the support member 350 is assembled in the key member 331 and the decoration part 321 of the electronic device 10 according to this disclosure.

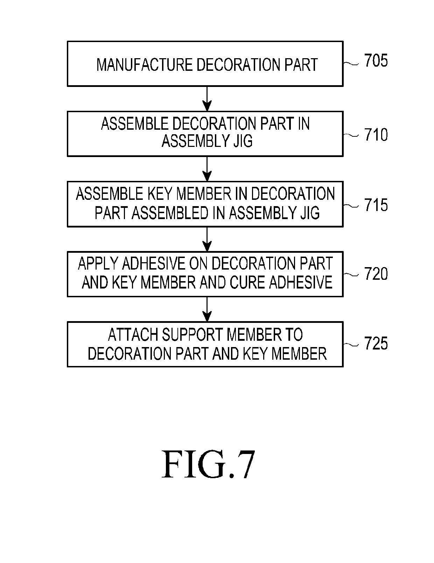

FIG. 7 is an example flowchart showing a method of manufacturing the home key 11a according to this disclosure.

The method of manufacturing the home key 11 a will be described with reference to FIG. 7. In operation 705, the decoration part 321 of the home key 11a can be manufactured.

In an embodiment, the decoration part of the home key 11a can be a metallic material or a nonmetallic material.

When the decoration part is formed of a metallic material, the metallic material can be zinc, magnesium, aluminum, SUS, tungsten, or the like and the decoration part can be manufactured through die casting, pressing, NC machining, or the like. However, the present disclosure is not limited thereto, and can employ other materials and other methods.

When the decoration part is formed of a nonmetallic material, the nonmetallic material can be PC, PA, PPA, plating ABS, or the like, and the decoration part can be manufactured through injection-molding, insert injection-molding, dual injection-molding, NC machining, and the like. However, the present disclosure is not limited thereto, and can employ other materials and other methods.

In operation 710, for example after operation 705, the manufactured decoration part 321 can be overturned and assembled in an assembly jig 340, to which an air-suction system having a plurality of air suction pipes can be applied and can be fixed by the air suction pipes 341 at the same time.

For example, as shown in FIG. 6A, the decoration part 321 can be overturned such that an upper surface of the decoration part 321 faces a lower surface of the assembly jig 340. Then, the air suction pipes 341 formed on a lower surface of the assembly jig 340 can fix the decoration part 321 by using an air (306) suction force of the air-suction system.

In operation 715, for example after operation 710, the key member 331 of the fingerprint recognition sensor 330 can be overturned and assembled in the overturned decoration part 321 and the decoration part 321 and the key member 331 can be combined by the air suction pipes 341.

For example, as shown in FIG. 6B, the key member 331 can be overturned and coupled to an inside of the decoration part 321. The air suction pipes 341 can pull the key member 331 and the decoration part 321 with the same force by using the air (306) suction force of the air-suction system, and the assembly jig can fix the key member and the decoration part to the same location. Through this, an assembly tolerance generated when the air-suction system is not used can be minimized.

For example, the decoration part 321 and the key member 331 can minimize a height difference between an upper end surface of the decoration part and an upper end surface of the key member by using the air suction pipes 341 of the air-suction system.

In operation 720, for example after operation 715, an adhesive can be applied and cured on the decoration part 321 and an upper end surface of the key member 331 to attach the support member 350.

In operation 725, for example after operation 720, the support member 350 can be attached to upper portions of the decoration part 321 and the key member 331 by using an adhesive.

For example, as shown in FIG. 6C, the decoration part 321 and the key member 331 can reduce a height difference between an upper end surface of the decoration part of the home key 11a and an upper end surface of the key member 331 by attaching the support member 350 to the decoration part 321 and the key member 331 while a tolerance due to the height difference can be minimized, and accordingly, an assembly tolerance can be generated when the decoration part 321 and the key member 331 are assembled.

The key member 331 can include a key actuator 331a. The shape of the support member 350 can be formed to correspond to the shape of the key actuator 331a.

The home key 11a can be separated from the assembly jig 340 and can be overturned again at the same time.

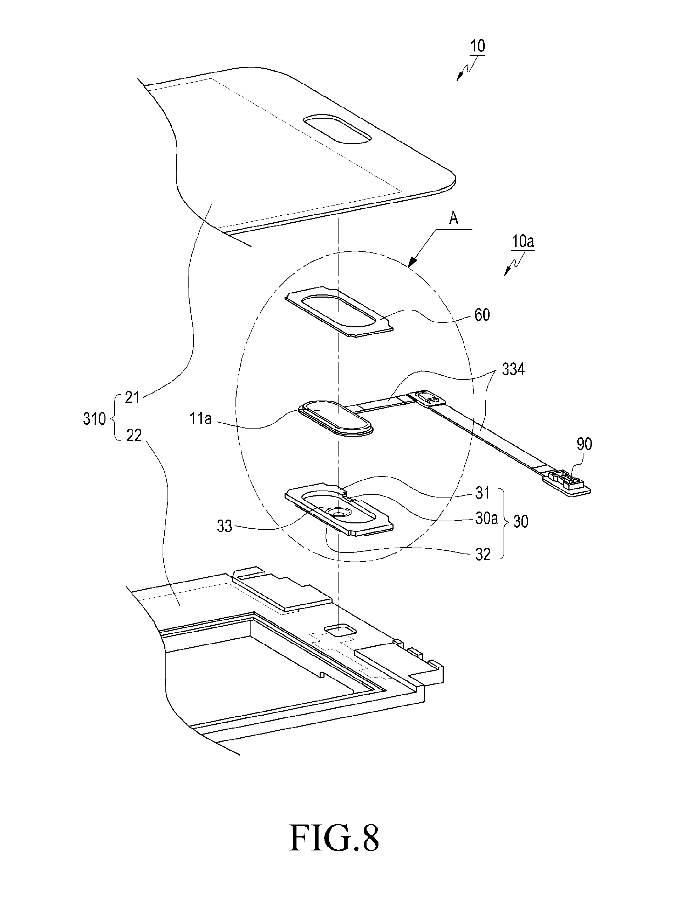

Hereinafter, the electronic device will be described with reference to FIGS. 8 to 16. In a description of the electronic device a repeated configuration thereof will be omitted and only a difference between the electronic device 10 (see FIG. 1) including the home key 11a (see FIG. 1) having the fingerprint recognition sensor 11a (see FIG. 1) will be described.

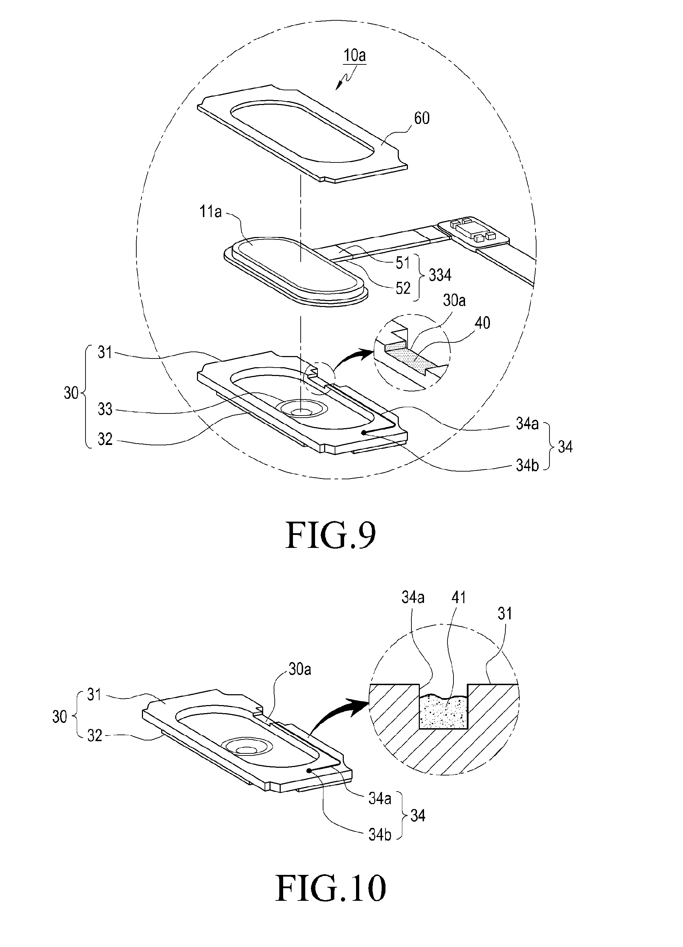

First, FIG. 8 is an example exploded perspective view showing a configuration of the electronic device 10 including a waterproof structure 10a according to this disclosure, and FIG. 9 is an example enlarged exploded perspective view of portion A of FIG. 8 according to this disclosure.

A configuration of the waterproof structure 10a for waterproofing the electronic device 10 will be described with reference to FIGS. 8 and 9. In an embodiment, the waterproof structure 10a can include a housing 310, a sealing member 30, a first bonding part 40, and an FPCB 334 or a second bonding part 60.

The housing 310 can include a window 21 and a bracket 22, and the sealing member 30 can be located between the bracket 22 and the window 21 of the housing 310 such that the FPCB 334 can pass between the bracket 22 and the window 21 of the housing 310. That is, the sealing member 30 can be assembled in the window 21 first. A through-portion 30a through which the FPCB 334 passes can be provided on an upper end surface of the sealing member 30. The first bonding part 40 can be provided in the through-portion 30a to be bonded to a rear surface of the FPCB 334. The FPCB 334 can be attached to the first bonding part 40 and can pass through the through-portion 30a of the sealing member 30. The second bonding part 60 can be provided between the window 21 and the sealing member 30 such that a front surface of the second bonding part 60 is bonded to a rear surface of the window 21 and a rear surface of the second bonding part 60 is attached to an upper surface of the sealing member 30 and is bonded to the front surface of the FPCB 334 at the same time.

For example, the front surface 51 of the FPCB 334 can be attached to a rear surface of the window 21 of the housing 310 by the second bonding part 60, and the rear surface 52 of the FPCB 334 can be bonded to the through-portion 30a by the first bonding part 40.

A connector 90 can be provided at one end of the FPCB 334 and any one of a home key 11a, a key button, a fingerprint recognition sensor, a connector 90, a printed circuit board (PCB) 80, a detection sensor, an optical sensor, a temperature sensor, a vein sensor, a human body detection sensor, and a living body recognition sensor can be provided at an opposite end of the FPCB 334.

Although the disclosed components are exemplified in the embodiment of the present disclosure, the present disclosure is not limited thereto. That is, any component which can be provided at opposite ends of the FPCB 334 can be applied as a modification. For example, the components can include components of all electronic devices electrically connected by the FPCB 334.

In an embodiment, the FPCB 334 can be attached to the through-portion 30a of the sealing member 30 and the window 21 of the housing 310 by using the first and second bonding parts 40 and 60, moisture introduced into a gap of the home key 11 a can be collected in an interior of the sealing member 30, a waterproof function of the electronic device can be realized by preventing the moisture collected in the sealing member 30 from penetrating into an electronic device through the through-portion 30a through which the FPCB 334 passes to be coupled to the through-portion 30a, and the FPCB 334 generated by a high temperature during an insert injection-molding can be prevented from being damaged when the FPCB 334 is integrally realized through insert injection-molding without using an existing insert injection-molding process and a separate waterproof member.

The sealing member 30 can have a U shape one side of which is opened. Although the sealing member can have a U shape, the present disclosure is not limited thereto. That is, any shape other than the U shape can be applied to the sealing member. For example, a box shape, one side of which is opened, can be applied to the sealing member 30.

The sealing member 30 can include a sealing bracket 31 and a resilient body 32, and the sealing bracket 31 can include a through-portion 30a coupled to the FPCB 334.

The resilient body 32 can be provided at a lower portion of the sealing bracket 31 to be pressed by resiliency when the FPCB 334 is coupled to the through-portion 30a formed in the sealing bracket 31.

In an embodiment, an actuator 33 can be provided in the sealing member 30 to face the key switch 81 provided in the printed circuit board 80 of the electronic device.

For example, the actuator 33 can face the home key 11a, and when the home key 11a is pressed, the actuator 33 can provide a click feeling and can press the key switch 81. Then, the key switch 81 can be electrically connected to the home key 11a to operate the home key 11a.

The sealing member 30 can be formed of any one of rubber, silicon, and the like and the sealing member 30 can be formed of a combination of dissimilar materials such as rubber, silicon, and the like. In an embodiment, while the sealing member can be formed of rubber, silicon, or the like, the present disclosure is not limited thereto. That is, the sealing member 30 can be formed of various materials such as a resilient material, a plastic such as PC, a metallic material such as SUS, and the like. For example, the first and second bonding parts 40 and 60 can be formed of an adhesive or a double-sided tape. The first bonding part 40 can be formed of an adhesive 41 and the second bonding part 60 can be formed of a double-sided tape or an adhesive.

That is, the through-portion 30a of the sealing member 30 can correspond to a bonding groove having a bonding space, and the first bonding part 40 formed of an adhesive 41 can be applied to the bonding groove. A rear surface of the FPCB 334 can be coupled and attached to the through-portion 30a to which the adhesive 41 can be applied to close the through-portion 30a.

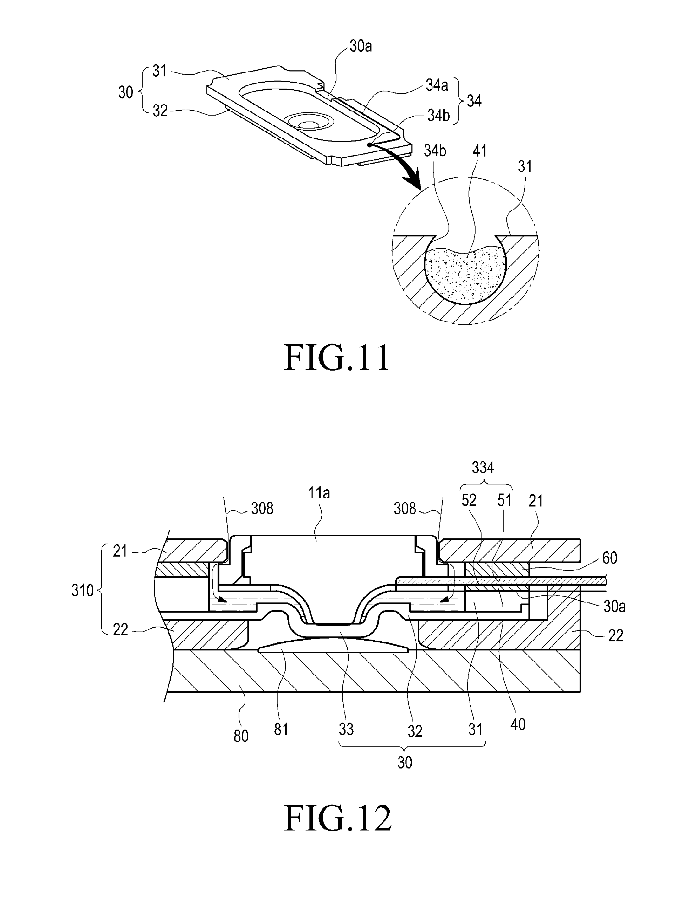

, FIG. 10 is an example of an enlarged side sectional view showing a movement passage 34a of the movement part 34 of the waterproof structure according to this disclosure. FIG. 11 is an example of an enlarged side sectional view showing a movement positioning part 34b of the waterproof structure 34 according to this disclosure.

Referring to FIGS. 10 and 11, because the moving part 34 for guiding and moving the adhesive 41 flowing to an outer edge of the through-portion 30a after the first bonding part 40 formed of the adhesive 41 can be applied to the through-portion 30a and can be provided in the sealing member 30, the adhesive 41 flowing to the outer edge of the through-portion 30a can be moved to the movement part 34 if a rear surface of the FPCB 334 is coupled to the through-portion 30a to close the through-portion 30a.

Because the movement passage 34a connected to the through-portion 30a to move the flowing adhesive 41 can be provided at one end of the movement part 34, the movement passage 34a can guide and move the adhesive 41 flowing to an outer edge of the through-portion 30a.

Because the movement positioning part 34b connected to the movement passage 34a to position the adhesive 41 moving through the movement passage 34a can be provided at an opposite end of the movement part 34, the movement positioning part 34b can position the adhesive 41 moving through the movement passage 34a and can restrain movement of the adhesive 41.

As shown in FIGS. 10 and 11, the movement passage 34a can correspond to a movement groove, and the movement positioning part 34b can correspond to a movement positioning groove.

In this way, because the movement part 34 can collect the adhesive 41 flowing to an outer edge of the through-portion 30a, it can be prevented from contacting other components.

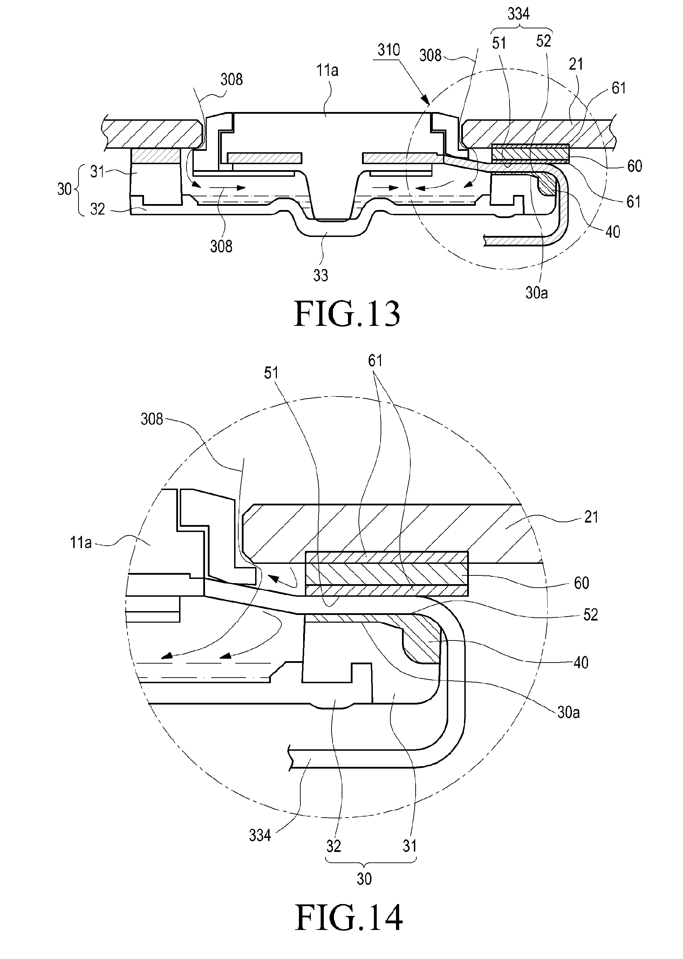

Referring to FIGS. 8 and 9, the first bonding part 40 including the adhesive 41 can be applied to the through-portion formed at an upper portion of the sealing member 30 first. A rear surface of the FPCB 334 can pass through an upper portion of the through-portion 30a to be coupled to the through-portion 30a and can be attached to the through-portion 30a by the first bonding part 40. Because one end of the FPCB 334 can be provided with the connector 90 and an opposite end of the FPCB 334 can be provided with the home key 11a, the home key 11a of the FPCB 334 can be coupled to an inside of the sealing member 30. The second bonding part 60 formed of a double-sided tape can be attached to an upper surface of the sealing member 30 while the FPCB 334 can be coupled to the through-portion 30a. Because an adhesive 61 (see FIG. 13) can be applied to upper and lower end surfaces of the second bonding part 60, the second bonding part 60 can be attached to a front surface of the FPCB 334 by the adhesive 61 (see FIG. 13) applied to a lower end surface of the second bonding part 60.

A rear surface of the FPCB 334 can be attached to the through-portion 30a by the first bonding part 40 formed of the adhesive 41, and a front surface of the FPCB 334 can be attached by the second bonding part 60 formed of a double-sided tape.

The home key 11a can be provided in the sealing member 30, and the FPCB 334 of the home key 11a can be sealed, waterproofed, and modulated into an independent component by using the first and second bonding parts.

The modulated home key 11a can be provided between the bracket 22 and the window 21 of the housing 310. Then, the actuator 33 provided in the sealing member 30 can face the key switch 81 provided in the printed circuit board 80.

The window 21 of the housing 310 can be attached to an upper end surface of the second bonding part 60 attached to the sealing member 30.

That is, because the adhesive 61 is applied to the upper end surface of the second bonding part 60 formed of a double-sided tape, the adhesive 61 of the second bonding part 60 can be bonded to a lower surface of the window 21.

FIG. 12 is an example side sectional view showing a coupled state of the waterproof structure according to this disclosure. FIG. 13 is an example side sectional view showing an in-use state of the waterproof structure according to this disclosure. FIG. 14 is an example of an enlarged side sectional view of part 310 of FIG. 11 according to this disclosure.

Referring to FIG. 12, the home key 11a can be coupled to an inside of the sealing member 30 and the FPCB 334 can be attached in stack by using the first and second bonding parts 40 and 60 to be assembled in the housing 310 of the electronic device in module.

As shown in FIGS. 13 and 14, if moisture 308 penetrates into a gap between the home key 11a and the window 21 of the housing 310, the moisture 308 can be collected in the interior of the sealing member 30 having a U-shape. Because the interior of the sealing member 30 can be sealed and waterproofed, the moisture 308 cannot be discharged to the outside of the sealing member 30.

Because both the front surface and the rear surface of the FPCB 334 of the home key 11a can be sealed by the first and second bonding parts 40 and 60, the moisture 308 introduced into the sealing member 30 cannot be introduced into the electronic device through the FPCB 334.

In an embodiment, the FPCB 334 can be attached in stack by using the first and second bonding parts 40 and 60 without using an existing insert injection-molding process and a separate waterproof member, the waterproof function of the FPCB 334 can be realized and damage of the FPCB 334 generated due to a high temperature during the insert injection-molding process can be prevented. In addition, manufacturing costs can be reduced by excluding an additional insert injection-molding process and a waterproof member.

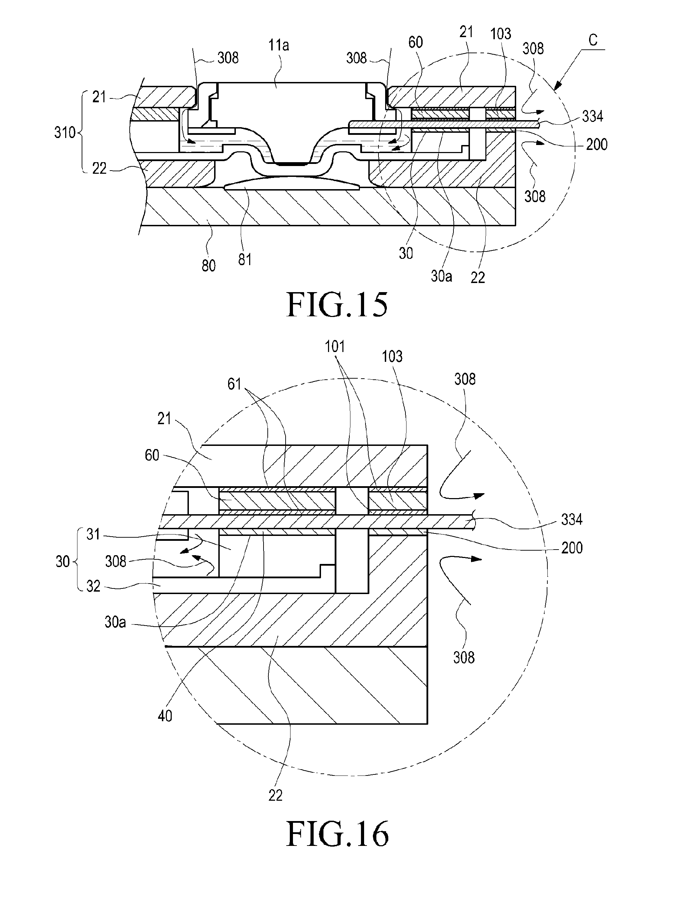

Hereinafter, the waterproof structure will be described with reference to FIGS. 15 to 16. In a description of the waterproof structure, it will be compared with the waterproof structure according to the above-described embodiment and only a difference between the embodiments will be described while a repeated configuration is omitted.

FIG. 15 is an example side sectional view showing a configuration of the waterproof structure according this disclosure, and FIG. 16 is an example of an enlarged side sectional view of portion 312 of FIG. 15 according to this disclosure. Referring to FIGS. 15 and 16, a configuration of the waterproof structure will be described.

The waterproof structure can include a housing 310 including a window 21 and a bracket 22, a sealing member 30, a first bonding part 40, an FPCB 334, a second bonding part 60, a third bonding part 103, and a fourth bonding part 200.

A through-portion 30a through which the FPCB 334 passes can be provided in the sealing member 30. The first bonding part 40 can be provided in the through-portion 30a to be bonded to a rear surface of the FPCB 334. The FPCB 334 can be attached to the first bonding part 40 and can pass through the through-portion 30a of the sealing member 30.

The second bonding part 60 can be provided between the window 21 and the sealing member 30 such that a front surface of the second bonding part 60 is attached to a rear surface of the window 21, and a rear surface of the second bonding part 60 is attached to an upper surface of the sealing member 30 and is bonded to the front surface of the FPCB 334 at the same time.

The third bonding part 103 can be provided between the window 21 and the bracket 22 to block the moisture 308 from penetrating from the outside of the housing 310.

The fourth bonding part 200 can be provided between the bracket 22 and the FPCB 334 to block the moisture Alfrom penetrating between the bracket 22 and the FPCB 334.

A connector 90 can be provided at one end of the FPCB 334, and a home key 11a can be provided at an opposite end of the FPCB 334.

In an embodiment, by attaching the FPCB 334 to the through-portion 30a of the sealing member 30 and the window 21 of the housing 310 in stack by using the first and second bonding parts 40 and 60 and sealing the interior of the home key 11 a, the moisture collected in the sealing member 30 can be prevented from penetrating into the electronic device through the through-portion 30a, into which the FPCB 334 penetrates to be coupled to the FPCB 334, making it possible to realize the waterproof function of the electronic device. In addition, the third and fourth bonding parts 103 and 200 can be additionally provided between the window 21 and the bracket 22, or between the bracket 22 and the FPCB 334 to prevent the introduced moisture 308 from penetrating to the outside of the housing 310, so that primarily, the moisture 308 in the sealing member 30 can be prevented from penetrating into the electronic device and secondarily, the moisture 308 penetrating can be prevented from the outside of the housing 310, making it possible to further improve the waterproof function of the electronic device.

For example, the third and fourth bonding parts 103 and 200 can be formed of an adhesive or a double-sided tape. The third bonding part 103 can be formed of a double-sided tape and the fourth bonding part 200 can be formed of an adhesive.

A detailed description of the assembly of the waterproof structure according to the other one or more embodiments can be the same as that of the waterproof structure as described herein, and will be omitted.

The modulated home key 11a can be provided between the bracket 22 and the window 21 of the housing 310. The FPCB 334 passing through and protruding from the through-portion 30a of the sealing member 30 can be bonded to the bracket 22 by the fourth bonding part 200 formed of an adhesive applied to the bracket 22. The window 21 of the housing 310 can be attached to an upper end surface of the second bonding part 60, which is attached to the sealing member 30. Because the adhesive 61 is applied to the upper end surface of the second bonding part 60 formed of a double-sided tape, the adhesive 61 of the second bonding part 60 can be bonded to a lower surface of the window 21. The third bonding part 103 provided on an upper surface of the FPCB 334 and an upper surface of the bracket 22 other than the FPCB 334 can attach the window 21 to the bracket 22 and the FPCB 334. The adhesive 101 provided on the upper surface of the third bonding part 103 can be attached to the lower surface of the window 21, and the adhesive 101 provided on the lower surface of the third bonding part 103 can be attached to the upper surface of the bracket 22 and the upper surface of the FPCB 334. That is, the third bonding part 103 can seal the lower surface of the window 21, seal the upper surface of the bracket 22 and the upper surface of the FPCB 334 and the fourth bonding part 200 can seal the bracket 22 and the FPCB 334. Thus, the third and fourth bonding parts 103 and 200 can block the moisture 308 from penetrating from the outside of the housing 310 to additionally realize the waterproof function.

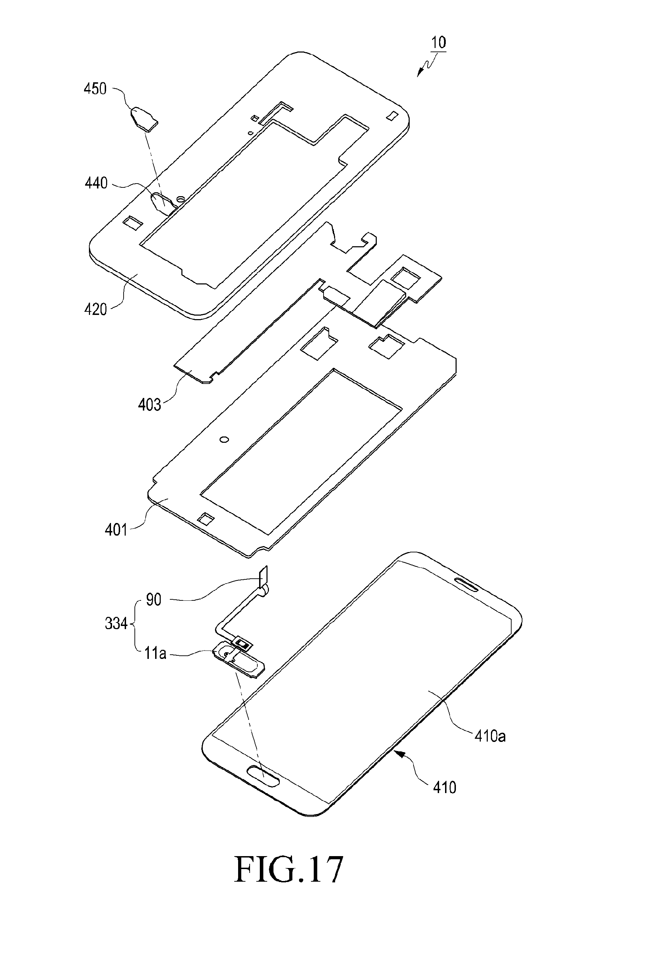

Hereinafter, the electronic device 10 will be described with reference to FIGS. 17 to 21. The electronic device will be compared with the electronic device structure according to other embodiments disclosed herein, and only a difference between the embodiments will be described while a repeated configuration is omitted.

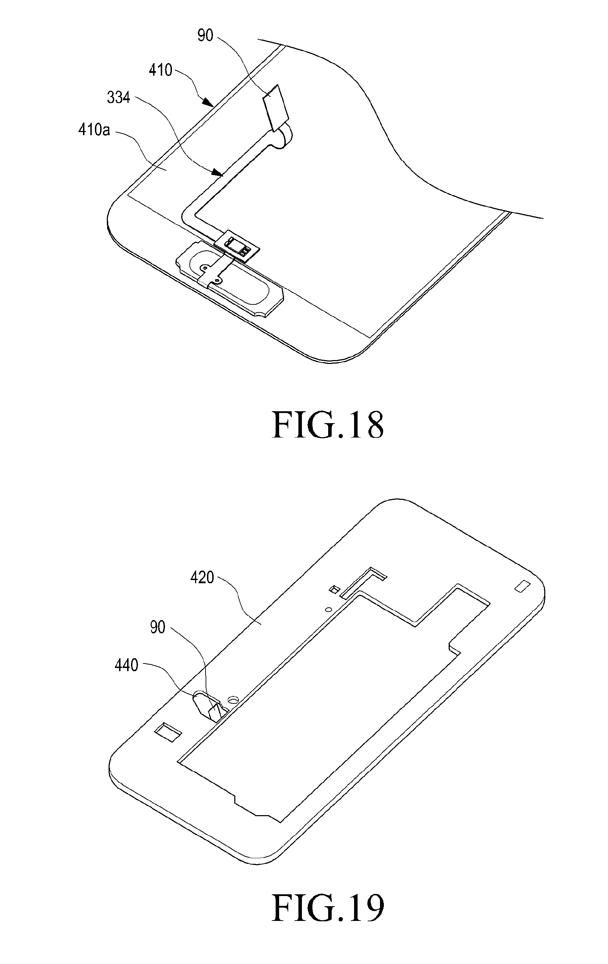

First, FIG. 17 is an example exploded perspective view showing configurations of the electronic device 10 according to this disclosure. FIG. 18 is an example perspective view showing a state in which the FPCB 334 of the electronic device 10 is coupled to a display module 410a including a window of the electronic device 10 according to this disclosure. FIG. 19 is an example perspective view showing a coupled state of the electronic device 10 according to this disclosure.

Referring to FIGS. 17 to 19, a configuration of the electronic device 10 will be described. The electronic device 10 can include a housing 410, a case 420, an FPCB 334, an opening/closing part 440, and a cap 450. The housing 410 can be sequentially coupled to the window 410a, the bracket 401, the printed circuit board 403 of the electronic device 10, and the case 420. The case 420 can be coupled to the housing 410 to include the opening/closing part 440. A connector 90 electrically connected to a connecting terminal 403a provided in the printed circuit board 403 can be provided at one end of the FPCB 334, and a home key 11a provided on one surface of the housing 410 can be provided at an opposite end of the FPCB 334. The FPCB 334 can be provided between the housing 410 and the case 420. The cap 450 can be detachably coupled to the opening/closing part 440 to open and close the FPCB 334. A connector 90 can be provided at one end of the FPCB 334, and any one of a home key 11a, a key button, a fingerprint recognition sensor, a connector 90, a printed circuit board (PCB), a detection sensor, an optical sensor, a temperature sensor, a vein sensor, a human body detection sensor, and a living body recognition sensor can be provided at an opposite end of the FPCB 334.

Although the disclosed components are exemplified herein, the present disclosure is not limited thereto. That is, any component which can be provided at opposite ends of the FPCB 334 can be applied as a modification. For example, the components can include components of all electronic devices 10 electrically connected by the FPCB 334. Here, the home key 11a of the disclosed parts will be exemplified as an embodiment. In addition, the home key 11a can include a fingerprint recognition sensor.

In an embodiment, because the case 420 is provided with the opening/closing part 440 for opening and closing the connector 90 of the FPCB 334 and the cap 450, damage of the FPCB 334 generated when the electronic device 10 is disassembled can be prevented and the FPCB 334 can be replaced and reassembled without damage when it is replaced.

The housing 410 can include a display module 410a including a window, and the case 420 can be a rear case. The display module 410a and the rear case are exemplified as the housing 410 and the case 420, the present disclosure is not limited thereto. Various components other than the housing 410 and the case 420 can be applied. For example, the housing 410 and the case 420 can be formed of any one of an external housing, an external cover, a battery cover, and a front case.

The opening/closing part 440 can include a through-hole to open and close the FPCB 334. If the cap 450 is separated, the through-hole can open and close the connector 90 of the FPCB 334.

A waterproof structure can be provided in the FPCB 334 to waterproof the FPCB 334 provided in the home key 11a.

Here, the configuration of the waterproof structure can be the same as the waterproof structure shown in FIGS. 11 and 12.

The home key 11a can be assembled in the window 21, and the FPCB 334 of the home key 11a can waterproof the FPCB 334 and the window 21 by using the waterproof structure. That is, it can be identified that the FPCB 334 and the window 21 can be waterproofed while the window 21 and the FPCB 334 are assembled.

Here, a process of assembling and disassembling the FPCB 334 provided in the electronic device 10 will be described with reference to FIG. 17 to 19.

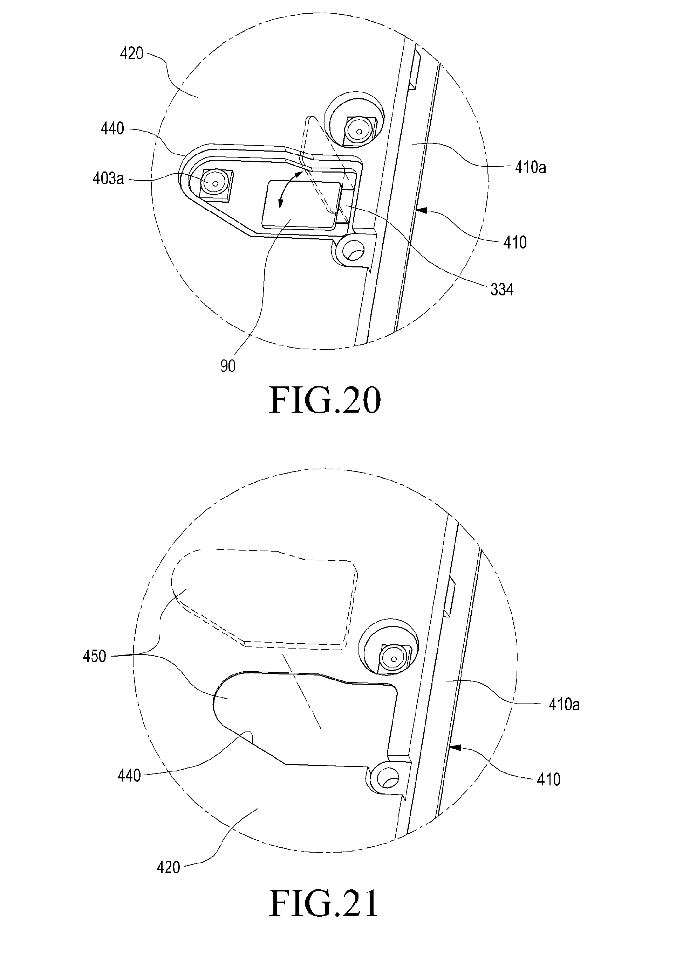

FIG. 20 is an example perspective view showing an operation of the opening/closing part 440 of the electronic device 10 according to this disclosure. FIG. 21 is an example perspective view showing an operational state of the cap 450 of the electronic device 10 according to this disclosure.

As shown in FIGS. 17 and 18, the home key 11a provided at one end of the FPCB 334 can be coupled to one surface of the window 410a of the housing 410. When viewed from an opposite surface of the one surface including the display, the printed circuit board 403 can be positioned on the case 420, and then the bracket 401 can be positioned, and finally, the connector 90 can be assembled on a rear surface of the window 410a after the window 410a is positioned.

As shown in FIG. 19 and 20, the connector 90 of the FPCB 334 can pass through, and can be coupled to the opening/closing part 440 of the case 420. The coupled connector 90 can be rotated to be electrically connected to the connecting terminal 403a provided in the printed circuit board 403.

As shown in FIG. 21, the cap 450 can be coupled to an upper portion of the opening/closing part 440 to close the opening/closing part 440.

The printed circuit board 403 can be assembled on one surface of the bracket 401 first, and can be assembled in the case 420 while the bracket 401 and the printed circuit board 403 are assembled. Finally, while the home key 11 a can be coupled to one end of the window 410a, the window 410a can be assembled in the case, and, finally, the assembling process can be finished by coupling the cap 450 to the opening/closing part 440 of the case 420.

When the assembled electronic device 10 is to be disassembled, first, the cap 450 coupled to the case 420 can be separated from the opening/closing part 440, and the opening/closing part 440 can be opened and the connector 90 of the FPCB 334 can be exposed together. In this state, the connector 90 can be separated from the connecting terminal 403a of the printed circuit board 403. Then, the display module including the window can be separated from the case, and the printed circuit board 403 and the bracket 401 assembled in the case can be separated from each other.

The FPCB 334 may not be damaged even if the window is separated from the case.

In this way, because the existing electronic device can be configured such that it is disassembled while the connector of the existing FPCB and the connecting terminal of the printed circuit board are electrically connected to each other, the FPCB can be damaged when the connector of the existing FPCB and the connecting terminal of the printed circuit board are separated from each other while they are coupled to each other and accordingly, they cannot be reassembled.

Accordingly, in order to overcome the disadvantages, the opening/closing part 440 for opening and closing the connector 90 of the FPCB 334 and the cap 450 can be provided in the case 420 of the electronic device 10, so that the cap 450 coupled to the opening/closing part 440 is separated first when the electronic device 10 is disassembled, and after the connector 90 of the FPCB 334 and the connecting terminal 403a of the printed circuit board 403 are separated from each other through the opened opening/closing part 440, the components of the electronic device 10 can be sequentially disassembled, and the FPCB 334 also can be disassembled without damage. Accordingly, the separated FPCB 334 can be reassembled without damage when the electronic device 10 is reassembled.

canAlthough the present disclosure has been described with an exemplary embodiment, various changes and modifications can be suggested to one skilled in the art. It is intended that the present disclosure encompass such changes and modifications as fall within the scope of the appended claims.

## Claims

1. An electronic device (10) comprising:
a housing (310);
a pressible key (11a) provided on one surface of the housing (310); and
a fingerprint recognition sensor (330) formed in the key (11a) to be at least partially exposed to a surface of the key (11a),
wherein an uppermost surface of the fingerprint recognition sensor (330) protrudes further than a surface of the housing (310) surrounding the key (11a).

2. The electronic device (10) of claim 1, wherein a protruding area (304) is formed between a front surface of the fingerprint recognition sensor (330) and the housing (310).

3. The electronic device (10) of claim 1, wherein the fingerprint recognition sensor (330) comprises a swipe type sensor.

4. The electronic device (10) of claim 1, wherein one surface of the housing (310) comprises:
a first side (1);
a second side (2) longer than the first side (1) and perpendicular to the first side (1);
a third side (3) having the same length as that of the first side (1) and parallel to the first side (1); and
a fourth side (4) having the same length as that of the second side (2) and parallel to the second side (2),
the fingerprint recognition sensor (330) having a length extending in a direction parallel to the third side (3) and perpendicular to the lengthwise direction, and the length is larger than the width.

5. The electronic device (10) of claim 1, further comprising a ring (321) at least partially surrounding the key (11a) when viewed from an upper side of one surface of the housing (310).

6. The electronic device (10) of claim 5, wherein the ring (321) comprises a metallic material and the ring (321) is electrically isolated from the fingerprint recognition sensor (330).

7. The electronic device (10) of claim 5, wherein the ring (321) has at least one plane, at least one curved surface, or a combination of at least one plane and at least one curved surface which extends from a surface of the housing (310) to an uppermost surface of the fingerprint recognition sensor (330).

8. The electronic device (10) of claim 1, wherein the fingerprint recognition sensor (330) comprises:
a key member (331);
a substrate (332a) provided on one side of the key member (331);
a signal pattern part (333) provided in the substrate (332a), for generating a transmission (Tx) signal or a reception (Rx) signal according to a fingerprint contact of a user; and
an FPCB (334) electrically connected to the signal pattern part (333).

9. The electronic device (10) of claim 8, wherein the signal pattern part (333) comprises:
a plurality of transmission (Tx) lines (333a); and
a plurality of reception (Rx) (333b) lines the number of which is smaller than that of the transmission (Tx) lines (333a).

10. The electronic device (10) of claim 1, wherein a coating layer (330a) is provided on an upper end surface of the fingerprint recognition sensor (330).

11. The electronic device (10) of claim 8, wherein a key actuator (331a) protrudes from a lower portion of the key member (331).

12. The electronic device (10) of claim 11, further comprising a support member (350) provided at a lower portion of the key (11a) and coupled to the key (11a) while surrounding a circumference of an outer edge of the key actuator (331a).

13. The electronic device (10) of claim 12, wherein a base member having a through-hole coupled to the key (11a) and through which the key actuator (331a) and the support member (350) pass to face a key switch (81) provided in the printed circuit board (80) is provided in the key (11a).

14. A method of manufacturing an electronic device (10), the method comprising:
manufacturing a decoration part (321) of a key(11a);
assembling the decoration part (321) in an assembly jig (340) to which an air-suction system having a plurality of air suction pipes (341) are applied after overturning the decoration part (321) and fixing the decoration part (321) by using the air suction pipes (341);
assembling a key member (331) of a fingerprint recognition sensor (330) in the overturned decoration part (321), and reducing an assembly tolerance between the decoration part (321) and the key member (331) by using the air suction pipes (341);
applying an adhesive to the decoration part (321) and an upper end surface of the key member (331); and
attaching a support member (350) to the decoration part (321) and an upper portion of the key member (331) by using the adhesive.

15. An electronic device (10) comprising:
a housing (310);
a through-hole (30a) formed at a portion of one surface of the housing (310);
a pressable key (11a) exposed through the through-hole (30a) of the housing (310);
a key switch (81) located within the housing (310) and configured to detect pressing of the key (11a);
a fingerprint recognition sensor (330) provided within the key (11a) and exposed to an upper surface of the key (11a); and
a flexible member inserted between the key (11a) and the key switch.

16. The electronic device (10) of claim 15, further comprising a waterproof structure (10a) extending from an inner surface of the housing (310) around the through-hole (30a) and connected to the flexible member to surround the key (11a) within the housing (310).

17. The electronic device (10) of claim 16, wherein the key (11a) further comprises a flexible printed circuit board (FPCB) (334) electrically connected to a sensor, and the FPCB (334) extends from a portion of the key (11a), and is disposed to pass through an opening formed in the waterproof structure (10a).

18. The electronic device (10) of claim 17, wherein the waterproof structure (10a) comprises:
a sealing member (30) located between the housing (310) and the window and including a through-portion (30a);
a first bonding part (40) provided in the through-portion;
the FPCB (334) attached to the first bonding part (40) and coupled to the through-portion (30a) of the sealing member (30); and
a second bonding part (60) provided between the housing (310) and the sealing member (30).

19. The electronic device (10) of claim 18, wherein the sealing member (30) has a box shape one surface of which is opened.

20. The electronic device (10) of claim 18, wherein any one of a key, a key button, a fingerprint sensor, a connector (90), a printed circuit board (80), a detection sensor, an optical sensor, a temperature sensor, a vein sensor, and a body detection sensor is provided at opposite ends of the FPCB.

21. An electronic device (10) comprising:
a housing (310);
a sealing member (30) located within the housing (310) and having a through-portion (30a);
a first bonding part (40) provided in the through-portion (30a);
a flexible printed circuit board (FPCB) (334) attached to the first bonding part (40) and coupled to the through-portion (30a) of the sealing member (30); and
a second bonding part (60) provided between the housing (310) and the sealing member (30),
wherein a front surface of the FPCB (334) is attached to the housing (310) by the second bonding part (60), and a rear surface of the FPCB (334) is attached to the through-portion (30a) by the first bonding part (40).

22. The electronic device (10) of claim 21, wherein the housing (310) comprises a window or a bracket.

23. The electronic device (10) of claim 21, wherein the sealing member (30) has a U shape one side of which is opened.

24. The electronic device (10) of claim 21, wherein the sealing member (30) comprises:
a sealing bracket (31) having a through-portion; and
a resilient body (32) provided at a lower portion of the sealing bracket (31).

25. The electronic device (10) of claim 21, wherein the through-portion (30a) is a through-groove having a bonding space, and the through-groove is provided on an upper surface of the sealing member (30).

26. The electronic device (10) of claim 21, wherein the sealing member (30) further comprises an actuator (33) facing a key switch (81) provided in the electronic device (10).

27. The electronic device (10) of claim 21, wherein the sealing member (30) comprises a movement part (34) for guiding and moving the adhesive (41) flowing from the through-portion (30a) after the first bonding part (40) comprising an adhesive is applied to the through-portion (30a).

28. The electronic device (10) of claim 27, wherein one end of the movement part (34) has a movement passage (34a) connected to the through-portion (30a) to move the adhesive (41), and an opposite end thereof has a movement positioning part (30a) connected to the movement passage (34a) to position an adhesive (41) moving through the movement passage (34a).

29. An electronic device (10) comprising:
a housing (310) comprising a window (21) and a bracket (22);
a sealing member (30) located within the housing (310) and having a through-portion (30a);
a first bonding part (40) provided in the through-portion (30a);
a flexible printed circuit board (FPCB) (334) attached to the first bonding part (40) and coupled to the through-portion (30a) of the sealing member (30);
a second bonding part (60) provided between the housing (310) and the sealing member (30);
a third bonding part (103) provided between the window (21) and the bracket (22); and
a fourth bonding part (200) provided between the bracket (22) and the FPCB (334).

30. The electronic device (10) of claim 29, wherein the third bonding part (103) is provided on an upper surface of the FPCB (334) and an upper surface of the bracket (22).

31. An electronic device (10) comprising:
a housing (410);
a case (420) coupled to the housing (410);
a flexible printed circuit board (FPCB) (334) provided between the housing (410) and the case (420);
an opening/closing part (440) provided in the case (420); and
a cap (450) detachably coupled to the opening/closing part (440), for opening and closing the FPCB (334).

32. The electronic device (10) of claim 31, wherein the housing (410) comprises a window (410a) and the case (420) is a rear case.

33. An electronic device (10) comprising:
a housing (310);
a case (420) coupled to the housing (310);
a flexible printed circuit board (FPCB) (334) provided between the housing (310) and the case (420) and having a connector at one end thereof and a key at an opposite end thereof;
an opening/closing part (440) provided in the case (420); and
a cap (450) detachably coupled to the opening/closing part, for opening and closing the connector.
